# EUROPEAN PATENT SPECIFICATION

(11) **EP 2 444 098 B1**
(45) Date of publication and mention of the grant of the patent: **05.06.2019**
(21) Application number: 10788601.2
(22) Date of filing: 17.06.2010
(51) Int. Cl.: A61K 36/896, A61K 36/88, A61K 36/78, A61K 36/70, A61K 36/534, A61K 36/489, A61K 36/484, A61K 36/355, A61K 36/34, A61K 36/288, A61K 36/282, A61K 36/232, A61K 36/315, A61K 36/19, A61K 36/185, A61K 36/11, A61P 31/16, A61K 31/12, A61P 11/00

(54) **TRADITIONAL CHINESE MEDICINE COMPOSITION FOR TREATING H1N1 SWINE INFLUENZA, PREPARATION METHOD AND USE THEREOF**
ZUSAMMENSETZUNG NACH ART DER TRADITIONELLEN CHINESISCHEN MEDIZIN ZUR BEHANDLUNG DER H1N1 SCHWEINEGRIPPE SOWIE HERSTELLUNGS- UND VERWENDUNGSVERFAHREN DAFÜR
COMPOSITION DE MÉDECINE CHINOISE TRADITIONNELLE DESTINÉE AU TRAITEMENT DE LA GRIPPE PORCINE H1N1, SA MÉTHODE DE PRÉPARATION ET SON UTILISATION

(30) Priority: 16.06.2009 CN 200910148341
(43) Date of publication of application: 25.04.2012
(73) Proprietor: Livzon Pharmaceutical Group Inc., Zhuhai, Guangdong 519020 (CN); National Engineering Research Center for Modernization of traditional Chinese Medicine, Zhuhai, Guangdong 519020 (CN)
(72) Inventor: TAO, Desheng, Zhuhai Guangdong 519020 (CN); ZENG, Yongqing, Zhuhai Guangdong 519020 (CN); CAO, Hui, Zhuhai Guangdong 519020 (CN); GUAN, Yi, Zhuhai Guangdong 519020 (CN)
(74) Representative: Schiweck Weinzierl Koch Patentanwälte Partnerschaft mbB
(86) International application number: PCT/CN2010/000884
(87) International publication number: WO 2010/145205

(56) References cited:
- EP-A1- 1 849 473
- CN-A- 1 528 419
- CN-A- 1 565 607
- CN-A- 1 803 179
- CN-A- 1 839 988
- CN-A- 101 695 536
- DATABASE WPI Week 201032 Thomson Scientific, London, GB; AN 2010-E79103 XP002712974, & CN 101 695 536 A (KOICHIRO YOSHINO) 21 April 2010 (2010-04-21)
- DATABASE WPI Week 200605 Thomson Scientific, London, GB; AN 2006-039852 XP002712975, & CN 1 565 607 A (SHANXI YABAO PHARM GROUP CO LTD) 19 January 2005 (2005-01-19)
- DATABASE WPI Week 200502 Thomson Scientific, London, GB; AN 2005-014163 XP002712976, & CN 1 528 419 A (TIANYITANG GROUP CO LTD ZHEJIANG PROV) 15 September 2004 (2004-09-15)

## Description

### TECHNICAL FIELD

The present invention relates to a traditional Chinese medicine composition for treating Swine Influenza (SI), preparation method and use thereof. Specifically, the present invention relates to a traditional Chinese medicine composition made from herbs for treating Swine Influenza, preparation method and use thereof.

### BACKGROUND TECHNOLOGIES

The pathogen of swine influenza is swine influenza virus (SIV), which is a member of the Orthomyxoviridae family. The virus is pleomorphic and approximately 100 nm in diameter and has an envelope with projecting glycoproteins on the surface which is usually called spike. There are two types of spike, namely hemagglutinin (HA) and neuramidinase (NA), both of which are main evidences to identify subtypes and strains of influenza virus. HA can agglutinate erythrocytes of some animals under favorable conditions, and be involved in recognizing and attaching to target cells during infection. NA can not only enable adherent virus to detach from erythrocyte, but also have virus released from the infected cell. Hemagglutinin antibodies of the same virus can prevent virus from attaching to cells and therefore play a key role in prevention of viral infection; while neuramidinase antibodies can inhibit viral infection to cells. In addition to HA and NA, virus proteins include matrix protein (M), nuclear protein (NP), polymerase (PA, PB1, PB2) and non-structural protein (NS).

Swine influenza is an acute infectious respiratory disease in pigs, characterized by sudden onset, cough, dyspnea, fever and rapid prognosis. Swine influenza is a respiratory disease in pigs caused by virus that is influenza A virus. It usually outbreaks in pigs, and is highly infectious but seldom leads to death. The high incidence of swine influenza occurs during autumn and winter though it can be transmitted all over the year. Swine influenza virus is usually recognized as influenza C virus, or one of influenza A virus subtypes, which may cause an outbreak of influenza in pigs. Humans are seldom infected by swine influenza virus under normal conditions.

There are many types of influenza A viruses, including H1N1, H1N2, H3N1, H3N2 and H2N3 subtypes, all of which can cause influenza A (H1N1) infection. Different from avian influenza, influenza A (H1N1) can spread among humans. It once occurred that humans were infected with A (H1N1) influenza but no cases had shown that influenza A (H1N1) can be transmitted among humans. In April of 2009, Mexico reported a case that influenza A (H1N1) was transmitted from person to person, where the patient was infected with influenza A (H1N1) virus. It was found during gene analysis that the gene comprises the genes derived from swine, chicken and Asian, European, and American human races. Patients with influenza A (H1N1) infection often suffer from high fever with body temperature above 39 °C, severe headache, muscle pain, cough, nasal obstruction, red eyes and other symptoms.

Swine influenza virus (SIV) is a type of orthomyxoviruses that can lead to endemic influenza in pigs. The novel lethal virus previously called swine influenza virus (SIV) was renamed to influenza A (H1N1) by World Health Organization on 30 April 2010.

Influenza A (H1N1) virus belongs to influenza A virus and is a strain of H1N1 subtype swine influenza virus. The virus comprises the ribonucleic acid gene fragments derived from avian influenza virus, swine influenza virus and human influenza virus. It also possesses properties of Asian and African swine influenza viruses.

In the natural state, the connection between AI and human influenza is achieved through intermediate hosts, such as mammalians including pig, horse, dolphin and the like. However, antigenic variation (antigenic shift and antigenic drift) happens in swine influenza virus constantly and its host range is broadened. Nowadays, the swine influenza virus is capable of infecting human directly. Swine influenza virus can break through the species barrier to infect human directly and even cause deaths, which thereby imparts new significance of public health to AIV. Recently, outbreaks of swine influenza epidemics have occurred in many countries, which greatly threaten human health and global economic security.

However, since a long time ago, the influenza viruses found in human only included H1, H2 and H3 subtypes, so the prevention of influenza have been targeted against these 3 subtypes of viruses and the antiviral medicaments sold on market are aimed to treat these diseases. Hence, human body is not immune to the current swine influenza virus and the medicaments for preventing and treating human influenza cannot be applied to prevention of swine influenza. When highly pathogenic swine influenza virus invades human body, fever, muscle soreness, chill and other symptoms will be caused which are more severe than those caused by influenza. Severe complications and even deaths may be caused.

At present, the predominant anti-influenza drugs mainly include neuraminidase inhibitors such as Zanamivir, Oseltamivir, and etc.; ion channel blockers such as Amantadine and Rimantadine, which are deemed as the preferred drugs for treating and preventing influenza; and nucleoside drugs such as triazole nucleoside, namely ribavirin that is also called virazole.

However, the above mentioned medicaments have shown some limitations as follows:
(1) Neuraminidase inhibitors can effectively inhibit Type A and B influenza viruses. But this kind of drug is relatively expensive and its popularization is therefore restricted. For example, Tamiflu, the generic name of which is Oseltamivirphosphate (CAS No. 204255-11-8), namely the phosphate of Oseltamivir, ethyl (3R,4R,5S)-4-acetylamino-5-amino-3(1-ethylpropoxy)-1-cyclohexene-1-carboxylate (CAS No. 196618-13-0), is exclusively produced by Roche Pharmaceutical Company. This medicine should be taken twice a day with a dosage of 75 mg per time, which means that it costs 60 RMB per day and approximately 300 RMB for a 5-day course of medicine treatment. Furthermore, Roche Pharmaceutical Company who possesses the Tamiflu patent till 2017 refuses to abandon the patent, on the grounds of complicated and time-consuming production technique, high quality standard, and etc. Thus, the yield of Tamiflu is not adequate and even in short supply.
   Moreover, there are significant side effects caused by Tamiflu, including obnubilation, hallucination, abnormal behavior and other psychological and neural symptoms. Drug resistance has also been observed in some of the patients.
(2) Ion channel blockers can effectively inhibit influenza A virus. However, it is toxic to nervous system and could induce drug resistance if taken for a long time which may lead to influenza pandemic. Additionally, it has no effect on influenza B virus.
(3) Triazole nucleoside (virazole) can effectively treat infections caused by RNA viruses or DNA viruses. However, this compound has some teratogenic effect which limits its application in clinic.

CN101695536 A and the Database WPI entry week 201032, Thomson Scientific, London, AN2010-E79103 discose a Chinese medicine composition for use in prevention and treatment of influenza A H1N1 virus.

CN1565607 A and the Database WPI entry week 200605, Thomson Scientific, London, AN2006-039852 disclose a Chinese medicinal composition for treating viral infection of upper respiratory tract.

CN1528419 A and the Database WPI entry week 200502, Thomson Scientific, London, AN2005-014163 disclose a Chinese medicine Kangbingdu pill preparation for resisting virus.

EP 1849473 A1 discloses a Chinese traditional medicine composition for treatment of avian influenza, method for preparation, and application thereof.

Thus, a more safe, effective, and cheaper drug of wide sources which can prevent and treat swine influenza effective appears to be more urgent and important.

In order to meet huge demand, many scientific and technical workers have focused their attention to Chinese traditional herbs. However, the traditional Chinese medicine compositions have little effects on treating swine influenza and therefore fail to meet the requirements to contain an outbreak of swine influenza quickly.

### SUMMARY OF THE INVENTION

Another objective of the present invention is to provide a preparation method for the traditional Chinese medicine compositions of the present invention.

Yet another objective of the present invention is to provide the uses of the traditional Chinese medicine compositions of the present invention in preparing drugs and/or health products which are used to prevent or treat swine influenza in human or animal, wherein the swine influenza is influenza A caused by H1N1.

According to the above-mentioned objectives, the present invention provides the embodiments as follows.

In one aspect, the present invention provides the use of a traditional Chinese medicine composition in preparing drugs and/or health products for preventing and treating swine influenza, active ingredients of which are substantially made from the following raw medicines: Herba Houttuyniae, Caulis Lonicerae Japonicae, Radix Isatidis, and Radix et Rhizoma Sophorae Tonkinensis, Rhizoma Dryopteris Crassirhizomatis Radix Angelicae Dahuricae preferably only made from the six above-mentioned raw medicines.

Preferably, the said traditional Chinese medicine composition also comprises other herbs for heat-clearing and detoxicating which are preferably selected from the group consisting of, , *Herba Andrographis, Herba Taraxaci, Indigo Naturalis, Caulis Sargentodoxae, Rhizoma Paridis, Herba Artemisiae Annuae, Rhizoma Iridis Tectori, Fructus Bruceae, Herba Menthae, Radix Platycodonis,* and *Radix Rhizoma Glycyrrhizae*; The weight percentages of the said raw materials of the said four active ingredients are Herba houttuyniae 4.2 wt. %-58.4wt. %, Caulis Lonicerae Japonicae 4.7 wt. %-46.7 wt. %, Radix Isatidis 2.3 wt. %-42.0 wt. %, *Radix et Rhizoma Sophorae Tonkinensis* 1.2 wt. %-35.0 wt. %, Rhizoma Dryopteris Crassirhizomatis 1.9 wt. %-37.3 wt. %, and Radix Angelicae Dahuricae 1.2 wt. %- 35.0 wt. %.

In another aspect, the present invention provides a traditional Chinese medicine composition for preventing or treating swine influenza, active ingredients of which are substantially made from the following raw materials: Herba Houttuyniae, Caulis Lonicerae Japonicae, Radix Isatidis, Radix et Rhizoma Sophorae Tonkinensis, Rhizoma Dryopteris Crassirhizomatis, Radix Angelicae Dahuricae, Rhizoma Iridis Tectori Herba Artemisiae Annuae, and Rhizoma Paridis preferably only made from the snine above-mentioned raw materials.

Preferably, the said traditional Chinese medicine composition also comprises other herbs for heat-clearing and detoxicating, which are preferably selected from the group consisting of *Herba Andrographis, Herba Taraxaci, Indigo Naturalis, Caulis Sargentodoxae,* , , , *Fructus Bruceae*, *Herba Menthae*, *Radix Platycodonis*, and *Radix Rhizoma Glycyrrhizae..*

The weight percentages of the said raw materials of the said four active ingredients are: Herba Houttuyniae 9.3 wt. %-46.7 wt. %, Caulis Lonicerae Japonicae 9.3 wt. %-37.3 wt. %, Radix Isatidis 4.7 wt. %-35.0 wt. %, Radix et Rhizoma Sophorae Tonkinensis 2.3 wt. %-23.3 wt. %, Rhizoma Dryopteris Crassirhizomatis 2.3 wt. %-28.0 wt. %, Radix Angelicae Dahuricae 2.3 wt. %- 23.3 wt. %., Rhizoma Paridis 2.3 wt. %-23.3 wt. %, Herba Artemisiae Annuae 2.3 wt. %-23.3 wt. %, and Rhizoma Iridis Tectori 2.3 wt. %-23.3 wt. %.

In yet another aspect, the present invention provides a traditional Chinese medicine composition for preventing or treating swine influenza comprising active ingredients and/or pharmaceutically acceptable carriers, the active ingredients of which are substantially made from the following raw materials: Herba Houttuyniae 14.0 wt. %-35.0 wt. %, Caulis Lonicerae Japonicae 11.7 wt. %-23.3 wt. %, Radix Isatidis 9.3 wt. %-21.0 wt. %, Rhizoma Dryopteris Crassirhizomatis 7.0 wt. %-18.7 wt. %, Radix et Rhizoma Sophorae Tonkinensis 4.7 wt. %-11.7 wt. %, Radix Angelicae Dahuricae 4.7 wt. %- 11.7wt. %, Rhizoma Paridis 4.7 wt. %-11.7 wt. %, Herba Artemisiae Annuae 4.7 wt. %-11.7 wt. %, and Rhizoma Iridis Tectori 4.7 wt. %-11.7wt. %.

Preferably, the said active ingredients are substantially made from the following raw materials: Herba Houttuyniae 21.4 wt. %, Caulis Lonicerae Japonicae 17.9 wt. %, Radix Isatidis 14.3 wt. %, Rhizoma Dryopteris Crassirhizomatis 10.7 %, Radix et Rhizoma Sophorae Tonkinensis 7.1 wt. %, Radix Angelicae Dahuricae 7.1 wt. %, Rhizoma Paridis 7.1 wt. %, Herba Artemisiae Annuae 7.1 wt. %, and Rhizoma Iridis Tectori 7.1 wt. %.

The traditional Chinese medicine compositions of the present invention can be made into any pharmaceutically acceptable dosage form, preferably into granule form.

In a further aspect, the present invention provides the use of said traditional Chinese medicine composition wherein the traditional Chinese medicine composition is prepared by a method comprising the following steps:
(1) All raw materials are weighed for use;
(2) Herba Houttuyniae is decocted in water for 10∼40 minutes and then filtered. The obtained filtrate is concentrated to obtain a clear paste with a relative density of 1.05 and then the paste is cooled down;
(3) Ethanol is added to the clear paste obtained in Step (2) until content of ethanol reaches 50∼80%. The mixture is left to stand for 24 hours and then filtered. After recovery of ethanol, the filtrate is concentrated to obtain a clear paste with a relative density of 1.25;
(4) The remaining raw materials are decocted in water twice, for 2 hours per time, and then filtered. The filtrate is concentrated until the relative density reaches 1.20 and then the concentrated filtrate is filtered and concentrated again to obtain a clear paste with a relative density of 1.10-1.25;
(5) The clear pastes obtained in Step (3) and Step (4) are mixed together and then concentrated continuously to obtain a thick paste with a relative density of or greater than 1.26, followed by adding pharmaceutical adjuvants.

The said swine influenza is influenza A (H1N1), which is caused by influenza A viruses selected from the following subtypes: H1N1.

The said swine influenza is human swine influenza or animal swine influenza.

In a still further aspect, it is disclosed herein a method of preventing or treating of swine influenza comprising administering an effective amount of the said traditional Chinese medicine compositions to a patient in need.

The said swine influenza is influenza A (H1N1), which is preferably caused by influenza A viruses selected from the following subtypes: H1N1, H1N2, H3N1, H3N2, and H2N3.

Preferably, the said swine influenza is human swine influenza or animal swine influenza.

Further it is disclosed herein a traditional Chinese medicine composition for preventing and treating swine influenza, comprising active ingredients and/or pharmaceutically acceptable carriers. The said active ingredients are substantially made from the following raw materials: *Herba Houttuyniae* 4.2 wt. %-70.0 wt. %, *Caulis Lonicerae Japonicae* 4.2 wt. %-46.7 wt. %, *Radix Isatidis* 2.3 wt. %-42.0 wt. %, *Radix et Rhizoma Sophorae Tonkinensis* 1.2 wt. %-35.0 wt. %, and other herbs for heat-clearing and detoxicating.

Preferably, the said herbs for heat-clearing and detoxicating are selected from *Rhizoma Dryopteris Crassirhizomatis, Radix Angelicae Dahuricae, Herba Andrographis, Herba Taraxaci*, *Indigo Naturalis*, *Caulis Sargentodoxae*, and *Radix Rhizoma Glycyrrhizae.*

Preferably, the said active ingredients are made from the following four raw materials: *Herba Houttuyniae* 4.2 wt. %-70.0 wt. %, *Caulis Lonicerae Japonicae* 4.2 wt. %-46.7 wt. %, *Radix Isatidis* 2.3 wt. %-42.0 wt. %, and *Radix et Rhizoma Sophorae Tonkinensis* 1.2 wt. %-35.0 wt. %.

In another aspect, the present invention provides the use of a traditional Chinese medicine composition in preparation of drugs and/or health-care products for preventing or treating swine influenza in human or animal, wherein the swine influenza is influenza A caused by H1N1, comprising active ingredients and/or pharmaceutically acceptable carriers. The said active ingredients are substantially made from the following raw materials: *Herba Houttuyniae* 4.7 wt. %-58.4 wt. %, *Caulis Lonicerae Japonicae* 4.7 wt. %-46.7 wt. %, *Radix Isatidis* 2.3 wt. %-42.0 wt. %, *Radix et Rhizoma Sophorae Tonkinensis* 1.2 wt. %-35.0 wt. %, *Rhizoma Dryopteris Crassirhizomatis* 1.9 wt. %-37.3 wt. %, *Radix Angelicae Dahuricae 1.2* wt. %- 35.0 wt. %, and other herbs for heat-clearing and detoxicating.

Preferably, the said herbs for heat-clearing and detoxicating are selected from *Rhizoma Paridis, Herba Artemisiae Annuae, Fructus Bruceae*, *Herba Andrographis*, *Herba Taraxaci*, *Herba Menthae*, *Radix Platycodonis*, and *Radix Rhizoma Glycyrrhizae.*

Preferably, the said active ingredients are made from the following six raw materials: *Herba Houttuyniae* 4.7 wt. %-58.4 wt. %, *Caulis Lonicerae Japonicae* 4.7 wt. %-46.7 wt. %, *Radix Isatidis* 2.3 wt. %-42.0 wt. %, *Radix et Rhizoma Sophorae Tonkinensis* 1.2 wt. %-35.0 wt. %, *Rhizoma Dryopteris Crassirhizomatis* 1.9 wt. %-37.3 wt. %, and *Radix Angelicae Dahuricae* 1.2 wt. %- 35.0 wt. %.

In yet another aspect, the present invention provides the use of a traditional Chinese medicine composition in preparation of drugs and/or health-care products for preventing or treatming swine influenza in human or animal, wherein the swine influenza is influenza A caused by H1N1 comprising active ingredients and/or pharmaceutically acceptable carriers. The said active ingredients are made from the following raw materials: *Herba Houttuyniae* 9.3 wt. %-46.7 wt. %, *Caulis Lonicerae Japonicae* 9.3 wt. %-37.3 wt. %, *Radix Isatidis* 4.7 wt. %-35.0 wt. %, *Rhizoma Dryopteris Crassirhizomatis* 2.3 wt. %-28.0 wt. %, *Radix et Rhizoma Sophorae Tonkinensis* 2.3 wt. %-23.3 wt. %, *Radix Angelicae Dahuricae* 2.3 wt. %- 23.3 wt. %, *Rhizoma Paridis* 2.3 wt. %-23.3 wt. %, *Herba Artemisiae Annuae* 2.3 wt. %-23.3 wt. %, and *Rhizoma Iridis Tectori* 2.3 wt. %-23.3 wt. %.

Preferably, the said active ingredients are made from the following raw materials: *Herba Houttuyniae* 14.0 wt. %-35.0 wt. %, *Caulis Lonicerae Japonicae* 11.7 wt. %-23.3 wt. %, *Radix Isatidis* 9.3 wt. %-21.0 wt. %, *Rhizoma Dryopteris Crassirhizomatis* 7.0 wt. %-18.7 wt. %, *Radix et Rhizoma Sophorae Tonkinensis 4.7* wt. %-11.7 wt. %, *Radix Angelicae Dahuricae* 4.7 wt. %-11.7 wt. %, *Rhizoma Paridis* 4.7 wt. %-11.7 wt. %, *Herba Artemisiae Annuae* 4.7 wt. %-11.7 wt. %, and *Rhizoma Iridis Tectori* 4.7 wt. %-11.7 wt. %.

Preferably, the said active ingredients are made from the following raw materials: *Herba Houttuyniae* 21.4 wt. %, *Caulis Lonicerae Japonicae* 17.9 wt. %, *Radix Isatidis* 14.3 wt. %, *Rhizoma Dryopteris Crassirhizomatis* 10.7 %, Radix et *Rhizoma Sophorae Tonkinensis* 7.1 wt. %, *Radix Angelicae Dahuricae* 7.1 wt. %, *Rhizoma Paridis* 7.1 wt. %, *Herba Artemisiae Annuae* 7.1 wt. %, and *Rhizoma Iridis Tectori* 7.1 wt. %.

More preferably, the traditional Chinese medicine composition of the present invention can be made into any pharmaceutically acceptable dosage form, preferably into granule form.

In a further aspect, the present invention provides the use of said traditional Chinese medicine composition wherein the traditional Chinese medicine composition is prepared by a method of preparing the above-mentioned traditional Chinese medicine compositions, comprising the following steps:
(1) All raw materials are weighed for use;
*(2) Herba Houttuyniae* is decocted in water for 10∼40 minutes and then filtered. The filtrate is concentrated to obtain a clear paste with a relative density of 1.05 and then cooled down;
(3) Ethanol is added to the clear paste obtained in Step (2) until the content of ethanol reaches 50∼80%. The mixture is left to stand for 24 hours and then filtered. After recovery of ethanol, the filtrate is concentrated to obtain a clear paste with a relative density of 1.25;
(4) The other raw materials are decocted in water twice, for 2 hours per time and then filtered. The filtrate is concentrated until the relative density reaches 1.20 and then the concentrated filtrate is filtered and concentrated again to obtain a clear paste with a relative density of 1.10-1.25;
(5) The clear paste obtained in Step (3) and Step (4) are mixed together and then concentrated continuously to obtain a thick paste with a relative density of or greater than 1.26, followed by adding pharmaceutical adjuvants.

The present invention also provides the use of the said traditional Chinese medicine compositions in preparing drugs for preventing or treating swine influenza.

The present invention also provides the use of the said traditional Chinese medicine compositions in preparing health products for preventing or treating swine influenza.

The said swine influenza is influenza A (H1N1). Preferably, the swine influenza is human swine influenza. And preferably, the swine influenza is animal swine influenza.

It is further disclosed herein a traditional Chinese medicine composition for preventing and treating swine influenza comprises active ingredients and/or pharmaceutically acceptable carriers and the said active ingredients are made from the following raw materials: *Herba Houttuyniae* 4.7 wt. %-70.0 wt. %, *Caulis Lonicerae Japonicae* 4.7 wt. %-46.7 wt. %, *Radix Isatidis* 2.3 wt. %-42.0 wt. %, and *Radix et Rhizoma Sophorae Tonkinensis* 1.2 wt. %-35.0 wt. %.

In order to be more effective, the said active ingredients of the above mentioned traditional Chinese medicine composition are made from the following raw materials: *Herba Houttuyniae* 14.0 wt. %-46.7 wt. %, *Caulis Lonicerae Japonicae* 11.7 wt. %-46.7 wt. %, *Radix Isatidis* 9.3 wt. %-28.0 wt. %, and *Radix et Rhizoma Sophorae Tonkinensis* 4.7 wt. %-21.0 wt. %.

Furthermore, it is found in clinic that the above-mentioned traditional Chinese medicine composition can be combined with other herbs for heat-clearing and detoxicating, such as *Herba Andrographis*, *Herba Taraxaci*, *Indigo Naturalis*, *Caulis Sargentodoxae*, *Herba Menthae*, *Radix Rhizoma Glycyrrhizae* and the like.

In another embodiment of the present invention, the traditional Chinese medicine composition used for preparation of drugs and/or health-care products for preventing or treating swine influenza in human or animal, wherein the swine influenza is influenza A caused by H1N1, comprises active ingredients and/or pharmaceutically acceptable carriers and the said active ingredients are made from the following raw materials: *Herba Houttuyniae* 4.7 wt. %-58.4 wt. %, *Caulis Lonicerae Japonicae 4.7* wt. %-46.7 wt. %, *Radix Isatidis* 2.3 wt. %-42.0 wt. %, *Radix et Rhizoma Sophorae Tonkinensis* 1.2 wt. %-35.0 wt. %, *Rhizoma Dryopteris Crassirhizomatis* 1.9 wt. %-37.3 wt. %, and *Radix Angelicae Dahuricae* 1.2 wt. %- 35.0 wt. %.

In order to be more effective, the said active ingredients of the above-mentioned traditional Chinese medicine composition are made from the following raw materials: *Herba Houttuyniae* 14.0 wt. %-35.0 wt. %, *Caulis Lonicerae Japonicae* 11.7 wt. %-23.3 wt. %, *Radix Isatidis* 9.3 wt. %-21.0 wt. %, *Rhizoma Dryopteris Crassirhizomatis* 7.0 wt. %-18.7 wt. %, *Radix et Rhizoma Sophorae Tonkinensis 4.7* wt. %-11.7 wt. %, *Radix Angelicae Dahuricae* 4.7 wt. %-11.7 wt. %.

Furthermore, it is found in clinic that the above-mentioned traditional Chinese medicine composition can be combined with other herbs for heat-clearing and detoxicating, such as *Fructus Bruceae*, *Herba Andrographis*, *Herba Taraxaci*, *Herba Menthae*, *Radix Platycodonis*, and the like.

In yet another embodiment of the present invention, the traditional Chinese medicine composition used for preparation of drugs and/or health-care products for preventing or treating swine influenza in human or animal, wherein the swine influenza is influenza A caused by H1N1, comprises active ingredients and/or pharmaceutically acceptable carriers and the said active ingredients are made from the following raw materials: *Herba Houttuyniae* 9.3 wt. %-46.7 wt. %, *Caulis Lonicerae Japonicae* 9.3 wt. %-37.3 wt. %, *Radix Isatidis* 4.7 wt. %-35.0 wt. %, *Rhizoma Dryopteris Crassirhizomatis* 2.3 wt. %-28.0 wt. %, *Radix et Rhizoma Sophorae Tonkinensis 2.3* wt. %-23.3 wt. %, *Radix Angelicae Dahuricae* 2.3 wt. %- 23.3 wt. %, *Rhizoma Paridis* 2.3 wt. %-23.3 wt. %, *Herba Artemisiae Annuae* 2.3 wt. %-23.3 wt. %, and *Rhizo ma Iridis Tectori* 2.3 wt. %-23.3 wt. %.

Preferably, the said active ingredients are made from the following raw materials: *Herba Houttuyniae* 14.0 wt. %-35.0 wt. %, *Caulis Lonicerae Japonicae* 11.7 wt. %-23.3 wt. %, *Radix Isatidis* 9.3 wt. %-21.0 wt. %, *Rhizoma Dryopteris Crassirhizomatis* 7.0 wt. %-18.7 wt. %, *Radix et Rhizoma Sophorae Tonkinensis* 4.7 wt. %-11.7 wt. %, *Radix Angelicae Dahuricae* 4.7 wt. %-11.7 wt. %, *Rhizoma Paridis* 4.7 wt. %-11.7 wt. %, *Herba Artemisiae Annuae* 4.7 wt. %-11.7 wt. %, and *Rhizoma Iridis Tectori* 4.7 wt. %-11.7 wt. %.

More preferably, the said active ingredients are made from the following raw materials: *Herba Houttuyniae* 21.4 wt. %, *Caulis Lonicerae Japonicae* 17.9 wt. %, *Radix Isatidis* 14.3 wt. %, *Rhizoma Dryopteris Crassirhizomatis* 10.7 %, *Radix et Rhizoma Sophorae Tonkinensis* 7.1 wt. %, *Radix Angelicae Dahuricae* 7.1 wt. %, *Rhizoma Paridis* 7.1 wt. %, *Herba Artemisiae Annuae* 7.1 wt. %, and *Rhizoma Iridis Tectori* 7.1 wt. %.

The traditional Chinese medicine compositions of the present invention can be made into any dosage form by conventional methods of Chinese medicine preparation. For example, the raw materials of the present invention can be ground into powder, mixed and taken with water. The said raw materials can also be decocted in water and the decoction is then concentrated. In order to maximize the effect of all the raw materials, *Herba Houttuyniae* can preferably be subjected to special extraction, for example, extraction with ethanol. However, these cannot be used to limit the protection scope of the present invention.

Preferablly, the traditional Chinese medicine composition as disclosed herein, comprising *Herba Houttuyniae*, *Caulis Lonicerae Japonicae*, *Radix Isatidis* and *Radix et Rhizoma Sophorae Tonkinensis* can be prepared by following the steps below:
Prescription amounts of the four above-mentioned raw materials are weighed for use. The Herba Houttuyniae is firstly decocted in water for 10∼40 minutes and then is filtered. The filtrate is concentrated to obtain a clear paste with a relative density of 1.05-1.20 and then cooled down. Ethanol is added to the clear paste until the content of ethanol reaches 50∼80%, followed by standing for 24∼48 hours and then filtration.

After recovery of ethanol, the filtrate is concentrated to obtain a clear paste with a relative density of 1.05-1.25. The other three raw materials, including *Caulis Lonicerae Japonicae*, *Radix Isatidis* and *Radix et Rhizoma Sophorae Tonkinensis,* are decocted in water twice, 2 hours per time and then filtered. The filtrate is concentrated until the relative density reaches 1.40. The concentrated filtration is then filtered and concentrated again to obtain a clear paste with a relative density of 1.45. The obtained *Herba Houttuyniae* clear paste is then added and mixed. The mixture is concentrated continuously to obtain a thick paste with a relative density greater than 1.10. Finally, the thick paste and fine sucrose powder are mixed in a ratio of 1:3∼8, and then granualated and subpackaged.

The raw materials in the above-mentioned preparation method can also be combined with *Rhizoma Dryopteris Crassirhizomatis* and *Radix Angelicae Dahuricae*, or further combined with *Herba Andrographis*, *Herba Taraxaci*, *Indigo Naturalis*, *Caulis Sargentodoxae* or *Radix Rhizoma Glycyrrhizae.* The detailed preparation steps are as follows:
Prescription amounts of *Herba Houttuyniae*, *Caulis Lonicerae Japonicae*, *Radix Isatidis*, *Radix et Rhizoma Sophorae Tonkinensis, Rhizoma Dryopteris Crassirhizomatis* and *Radix Angelicae Dahuricae* are obtained in. *Herba Houttuyniae* is decocted in water for 10∼40 minutes and then is filtered. The filtrate is concentrated to obtain a clear paste with a relative density of 1.20 and then cooled down. Ethanol is added to the clear paste until the content of ethanol reaches 50∼80%, followed by standing for 24∼48 hours and then filtration. After recovery of ethanol, the filtrate is concentrated to obtain a clear paste with a relative density of 1.25. Other five raw materials, such as *Caulis Lonicerae Japonicae* and etc. are decocted in water twice, 2 hours per time and then filtered. The filtrate is concentrated until the relative density reaches 1.08. The concentrated filtrate is filtered and concentrated again to obtain a clear paste with a relative density of 1.10. The *Herba Houttuyniae* clear paste is then added and mixed. The mixture is concentrated continuously to obtain a thick paste with a relative density greater than 1.26. Finally, the thick paste and fine sucrose powder are mixed in a ratio of 1:3∼8, granualated and subpackageed.

The traditional Chinese medicine composition of the present invention comprising *Herba Houttuyniae, Caulis Lonicerae Japonicae, Radix Isatidis, Radix et Rhizoma Sophorae Tonkinensis*, *Rhizoma Dryopteris Crassirhizomatis*, *Radix Angelicae Dahuricae*, *Rhizoma Paridis*, *Herba Artemisiae Annuae*, and *Rhizoma Iridis Tectori* can be prepared in the following steps:
Prescription amounts of all nine above-mentioned raw materials are weighed for use. *Herba Houttuyniae* is decocted in water for 10∼40 minutes and then filtered. The filtrate is concentrated to obtain a clear paste with a relative density of 1.05 and then cooled down. Ethanol is added to the clear paste until the content of ethanol reaches 50∼80%, followed by standing for 24 hours and then filteration. After recovery of ethanol, the filtrate is concentrated to obtain a clear paste with a relative density of 1.25. Other eight raw materials such as *Caulis Lonicerae Japonicae* and etc. are decocted in water twice, 2 hours per time, and then filtered. The filtrate is concentrated until the relative density reaches 1.20. The concentrated filtrated is then filtered and concentrated again to obtain a clear paste with a relative density of 1.10-1.25. The *Herba Houttuyniae* clear paste is then added and mixed. The mixture is concentrated continuously to obtain a thick paste with a relative density greater than 1.26. Finally, the thick paste and fine sucrose powder are mixed in a ratio of 1:3∼8, granualated and subpackaged.

One or more pharmaceutically acceptable carriers can be added to the traditional Chinese medicine compositions of the present invention, for example, conventional pharmaceutical adjuvants used in preparation of different dosage forms, including diluting agents, excipients, fillers, adhesive, humectants, disintegrants, absorption enhancers, surfactants, absorbing carriers, lubricants, and the like.

The traditional Chinese medicine compositions of the present invention can be administered via different routes including oral, injection or mucosal administration. The said medicine compositions can be made into tablet, capsule, powder, granule, pastille, suppository, oral liquid, sterile parenteral suspension or other drug forms; can also be made into small or large dose injection, lyophilized powder injection, sterile powder injection or other injection forms. All of the above-mentioned dosage forms can be prepared by using conventional methods in the art.

The traditional Chinese medicine composition of the present invention can be added to livestock and poultry feeds as an additive in order to strengthen their immunity against swine influenza.

Effective dose of the traditional Chinese medicine composition or additive of the present invention is 20-80 mg/kg body weight per day. The preferred effective dose is 30-65 mg/kg body weight per day.

The traditional Chinese medicine composition of the present invention takes natural plants as raw materials has significant effect on inhibiting swine influenza viruses. When made into drug or additive, it has advantages in low toxicity, strong effectness, safety and etc., which provides new approaches to preventing and treating swine influenza Thus, the traditional Chinese medicine composition of the present invention will be of significant social and economic value and promise wide application prospects

### BRIEF DESCRIPTION OF THE DRAWINGS

The embodiments of the present invention will be described in details hereinafter by referring to the figures, wherein:

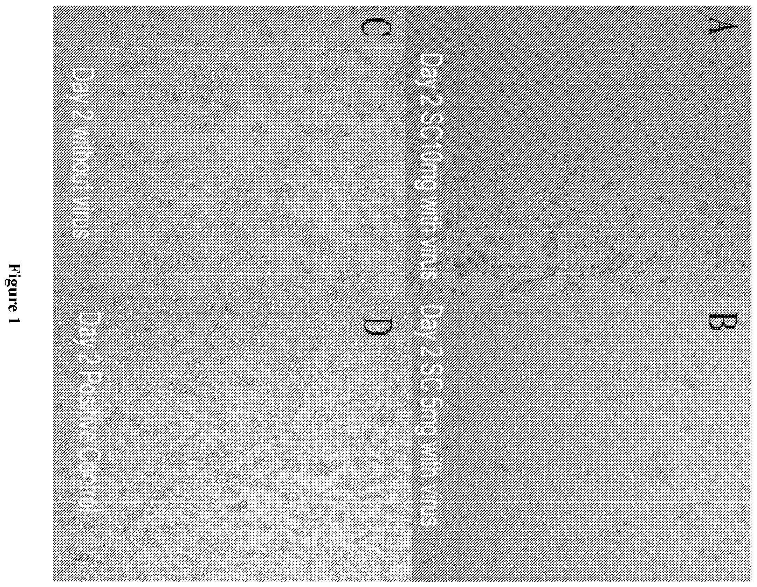

Figure 1 shows photographs of pathological changes of the cells on Day 2 of 3-day continuous observation during the *in vitro* research on the traditional Chinese medicine composition of the present invention against the swine influenza virus in Embodiment 22, wherein Picture A refers to the infected cells treated with 10 mg of the traditional Chinese medicine composition (SC 10 mg); Picture B refers to the infected cells treated with 5 mg of the traditional Chinese medicine composition (SC 5 mg); Picture C shows the negative control group, i.e., MDCK cells without being infected; Picture D shows the control group treated with 10 µmol/ml Tamiflu.

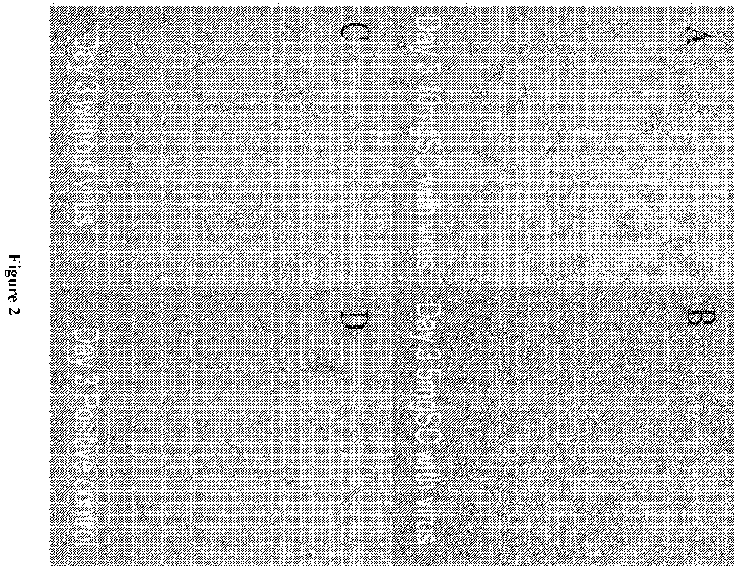

Figure 2 shows photographs of pathological changes of the cells on Day 3 of 3-day continuous observation during the *in vitro* research on the traditional Chinese medicine composition of the present invention against swine influenza virus in Embodiment 22, wherein Picture A shows the infected cells treated with 10 mg of the traditional Chinese medicine composition (SC 10 mg); Picture B shows the infected cells treated with 5 mg of the traditional Chinese medicine composition (SC 5 mg); Picture C shows the negative control group, i.e., MDCK cells without being infected; Picture D shows the control group infected cells treated with 10 µmol/ml Tamiflu.

### BEST MODES OF IMPLEMENTING THE PRESENT INVENTION

The present invention will be described further by combining the following embodiments.. With regard to those embodiments where the experimental conditions are not explicitly described, the experiments usually should be performed under the conventional conditions or under the conditions recommended by manufacturers.

The preparing method of the traditional Chinese medicine composition of the present invention is illustrated in the following Emboliments 1-19.

Unless specifically indicated, the raw materials, chemicals, and the equipments used in the following embodiments were purchased from the manufacturers known to those skilled in the art. For example, Herba Houttuyniae and Rhizoma Dryopteris Crassirhizomatis were purchased from South China Botanical Garden, located in Longdong, Guangzhou, Guangdong, China; Caulis Lonicerae Japonicae and Radix Isatidis were purchased from Medical Material Purchasing and Supply Station, located in Taihe, Anhui, China; Radix et Rhizoma Sophorae Tonkinensis, Radix Angelicae Dahuricae, Rhizoma Paridis, Herba Artemisiae Annuae, and Rhizoma Iridis Tectori were purchased from Medical Material Trading Center, located in Haozhou, Anhui, China.

### EMBODIMENT 1

### Preparation of Large Dose Injections

2000 g *Herba Houttuyniae*, 400 g *Caulis Lonicerae Japonicae*, 1500 g *Radix Isatidis*, 284 g *Rhizoma Dryopteris Crassirhizomatis*, 100 g *Radix et Rhizoma Sophorae Tonkinensis*, 100 g *Radix Angelicae Dahuricae*, 100 g *Rhizoma Paridis*, 100 g *Herba Artemisiae Annuae*, and 100 g *Rhizoma Iridis Tectori* were weighed and soaked in eightfold amount of water for 3 hours, then decocted and extracted twice for 1 hour per time. The extracts were pooled, filtered, and concentrated under reduced pressure until the relative density at 65 °C reached 1.25. It was then cooled down and mixed with ethanol until the content of ethanol reached 70%. After standing, the supernatant was collected. The ethanol was recovered and removed. The obtained solution was concentrated until the relative density at 65 °C reached 1.15 and then was extracted counter-current with double amount of ethyl acetate. The extracts were pooled and then the ethyl acetate was recovered and removed. A half of total amount of water for injection was added. The solution was boiled, cooled, filtered, and then supplemented with suitable amount of water for injection. 0.08% Tween-80 was added and mixed. And then the pH value was adjusted to 8.0 with 40% NaOH. NaCl was added and dissolved by stirring. A total amount of water for injection was added to obtain the isotonic solution. At last, the solution was filtered through a 0.22 µm microporous filtering film, followed by encapsulation, sterilization, lamp inspectation, and packaging.

### EMBODIMENT 2

### Preparation of Small Dose Injections

400 g *Herba Houttuyniae,* 1600 g *Caulis Lonicerae Japonicae,* 200 g *Radix Isatidis,* 100 g *Rhizoma Dryopteris Crassirhizomatis,* 1000 g *Radix et Rhizoma Sophorae Tonkinensis,* 200 g *Radix Angelicae Dahuricae*, 200 g *Rhizoma Paridis,* 384 g *Herba Artemisiae Annuae,* and 200 g *Rhizoma Iridis Tectori* were weighed and decocted in water and then filtered. The filtrate was concentrated under reduced pressure until 9 g raw medicine per ml was reached. The concentrate was then diluted with water, refrigerated, filtered, and concentrated under reduced pressure. The obtained concentrate was separated with macroporous resin which was washed with water, NaOH solution and 35 wt. % ethanol successively and then eluted with 75 wt % ethanol. The eluate was collected and diluted with water. L-Arginine was added to adjust the pH to 7.5, followed by adding active carbon. The solution was then heated and filtered to remove active carbon The filtrate was diluted with the same volume of water, followed by addition of L-Arginine until the concentration of L-Arginine reached 4 wt % to obtain the said small dose injection.

### EMBODIMENT 3

### Preparation of 20 ml Lyophilized Powder Injections

584 g *Herba Houttuyniae*, 500 g *Caulis Lonicerae Japonicae*, 400 g *Radix Isatidis*, 300 g *Rhizoma Dryopteris Crassirhizomatis*, 500 g *Radix et Rhizoma Sophorae Tonkinensis*, 500 g *Radix Angelicae Dahuricae*, 500 g *Rhizoma Paridis*, 500 g *Herba Artemisiae Annuae*, and 500 g *Rhizoma Iridis Tectori* were weighed and soaked in 3200 ml water for injection for 1 hour. 6400 ml water for injection was added and decocted twice for 1.5 hours per time. After filtering, the filtrate was pooled to obtain the extracts. 10 g of the extracts were dissolved with suitable amount of water for injection. After adding 2.25 g NaCl, water for injection was added until the volume reached 250 ml. Suitable amount of active carbon was added was and then boiled for 20 minutes, followed by cooling, filtration, and sterilization. The final solution was injected into low-borosilicate glass ampoules with 1ml per ampoules, and then dried by cooling to obtain lyophilized powder injection.

### EMBODIMENT 4

### Preparation of Small Dose Injection

200 g *Herba Houttuyniae*, 200 g *Caulis Lonicerae Japonicae*, 100 g *Radix Isatidis*, 1279 g *Rhizoma Dryopteris Crassirhizomatis*, 1500 g *Radix et Rhizoma Sophorae Tonkinensis*, 1500 g Radix *Angelicae Dahuricae* were weighed and decocted in water and then filtered. The filtrate was concentrated under reduced pressure Ethanol was then added followed by standing The solution was filtred and then the filtrate was concentrated under reduced pressure until 11 g raw medicine per ml was reached. The concentrate was diluted with water, refrigerated, filtered, and concentrated under reduced pressure. The obtained concentrate was separated with macroporous resin which was washed with water, NaOH solution and 55 wt. % ethanol successively, and then eluted with 65 wt. % ethanol. The eluate was collected and diluted with water. L-Arginine was then added to adjust the pH to 7.0, followed by adding active carbon. The solution was then heated and filtered to remove active carbon. The filtrate was diluted with the same volume of water to obtain the said small dose injection.

### EMBODIMENT 5

### Preparation of Granules

2500 g *Herba Houttuyniae*, 1000 g *Caulis Lonicerae Japonicae*, 400 g *Radix Isatidis*, 784 g *Rhizoma Dryopteris Crassirhizomatis*, 50 g *Radix et Rhizoma Sophorae Tonkinensis*, and 50 g *Radix Angelicae Dahuricae* were weighed and mixed with 70 wt. % ethanol in a multifunction extractor and then soaked overnight. Reflux extraction was performed twice for 1.5 hours per time. After filtration, dreg I and solution I were obtained. The solution I was transferred to a decompression pot, retrieved under reduced pressure until no ethanol was smelt, and then concentrated to obtain a thick extractum.

Starch was dried and sieved. Mannitol was also sieved for use. 500 g of the obtained thick extractum was mixed with 500 g mannitol and 150 g starch. The mixture was then made into granules in a granulator, dried on an ebullated bed and granulated with 12-mesh sieve. The content of granules and water content were examined. After that, the granules were packaged (7 g per bag) and sealed and then stored in a cool and dry place.

### EMBODIMENT 6 (not according to the present invention)

### Preparation Technique of Tablets

Firstly, 3000 g *Herba Houttuyniae*, 1134 g *Caulis Lonicerae Japonicae*, 100 g *Radix Isatidis*, and 50 g *Radix et Rhizoma Sophorae Tonkinensis* were weighed and soaked in 3200 ml water for injection for 1 hour. 3200 ml water for injection was added and then decocted twice for 1 hour per time. After filtering, the dregs were discarded while the obtained filtrate was concentrated in a triple-effect concentrator until the density reached 1.08 (70 °C) and then cooled to room temperature. The calculated amount of 95 wt. % ethanol was added to make the concentration of ethanol reach 50%, followed by standing overnight. The precipitates were discarded and the supernatant was collected and concentrated. The concentrate was then mixed with 200 g filler. The obtained mixture was placed in a granulator, added 60 ml of 30 wt. % ethanol and then granulated and dried.

Secondly, 50 g vitamins were mixed with 120 g filler. The obtained mixture was placed in a granulator, added 35 ml of 6 wt. % adhesive and then granulated and dried.

Thirdly, the two above-obtained kinds of granules were mixed in prescription proportion. After examining the contents, the mixture was pressed into tablets by 33-punching rotary tablet pressing machine and then subpackaged.

### EMBODIMENT 7

### Preparation Technique of Granules

918 g *Herba Houttuyniae*, 765 g *Caulis Lonicerae Japonicae*, 612 g *Radix Isatidis,* 459 g *Rhizoma Dryopteris Crassirhizomatis*, 306 g *Radix et Rhizoma Sophorae Tonkinensis*, 306 g *Radix Angelicae Dahuricae*, 306 g *Rhizoma Paridis*, 306 g *Herba Artemisiae Annuae*, and 306 g *Rhizoma Iridis Tectori* were weighed for use. The *Herba Houttuyniae* was firstly decocted in water for 40 minutes and then filtered. The filtrate was concentrated to obtain a clear paste with a relative density of 1.05 and then cooled downed. Ethanol was added to the paste until the amount of ethanol reaches 80% followed by standing for 48 hours and then filtered. After recovery of ethanol, the filtrate was concentrated to obtain a clear paste with a relative density of 1.25. Other eight raw materials were decocted in water twice for 2 hours per time and then filtered. The filtrate was concentrated until the relative density reached 1.08 and then filtered and concentrated again to obtain a clear paste with a relative density of 1.10. The above-obtained Herba Houttuyniae clear paste was then added and mixed. The mixture was concentrated continuously to obtain a thick paste with a relative density greater than 1.26. The thick paste and fine sucrose powder were mixed in a ratio of 1:5 and then granulated and subpackaged.

### EMBODIMENT 8

### Preparation Technique of Capsules

200 g *Herba Houttuyniae*, 500 g *Caulis Lonicerae Japonicae*, 384 g *Radix Isatidis*, 800 g *Rhizoma Dryopteris Crassirhizomatis*, 200 g *Radix et Rhizoma Sophorae Tonkinensis*, 200 g *Radix Angelicae Dahuricae*, 1000 g *Rhizoma Paridis*, 1000 g *Herba Artemisiae Annuae,* and 200 g *Rhizoma Iridis Tectori* were weighed and soaked in 3200 ml water for injection for 1 hour. 7200 ml water for injection was added and decocted twice for 1 hour per time. After filtering, the pooled filtrate was retrieved under reduced pressure until no ethanol was smelt and then concentrated to obtain a thick extractum with a relative density of 1.0 (60 °C). The extractum was then dried at 70 °C under reduced pressure for 36 hours in a vacuum drying oven, crushed and sieved with 60-80 mesh sieve.

The obtained powder was mixed with adjuvant, sieved and granulated. The granules were dried by heating, sieved with a 20 mesh sieve and then mixed with magnesium stearate. The obtained mixture was filled into capsules.

### EMBODIMENT 9 (not according to the present invention)

### Preparation Technique of Capsules

600 g *Herba Houttuyniae*, 2000 g *Caulis Lonicerae Japonicae*, 1200 g *Radix Isatidis*, and 484 g *Radix et Rhizoma Sophorae Tonkinensis* were weighed and soaked in 3200 ml water for injection for 1 hour. 3200 ml water for injection was added and decocted twice for 2.5 hours per time. After filtering, the pooled filtrate was recovered under reduced pressure until no ethanol was smelt and then concentrated to obtain a thick extractum with a relative density of 1.30. The extractum was then dried under reduced pressure at 80 °C for 48-96 hours in a vacuum drying oven, crushed and sieved with 60-80 mesh sieve.

The obtained powder was then mixed with adjuvant, sieved with a 60 mesh sieve and granulated. The granules were dried by heating, sieved and then mixed with magnesium stearate. The mixture was finally filled into capsules.

### EMBODIMENT 10 (not according to the present invention)

### Preparation of Health-care Foods (Dairy Products)

200 g *Herba Houttuyniae*, 2000 g *Caulis Lonicerae Japonicae*, 584 g *Radix Isatidis*, and 1500 g *Radix et Rhizoma Sophorae Tonkinensis* were soaked in 3200 ml water for injection for 1 hour. 3200 ml water for injection was added and then decocted twice for 2.5 hours per time. After filtering, the pooled filtrate was recovered under reduced pressure until no ethanol was smelt and then concentrated until the relative density reached 1.08.

Cow milk was inspected, purified by Alteration and processed according to standards (skipped in the case of skim milk). 0.5-6 mg of the above-obtained concentrate was added to each 100 g of the processed milk (comprising a suitable mount of flavoring agent). The solution was then filtered, preheated (≤115°C), sterilized, homogenized (skipped in the case of skim milk), cooled down, and then inoculated with fermentation strain (filling in advance if preparing set products). After fermentation, the obtained fermented milk was broken (skipped if preparing set products), cooled, filled (skipped if preparing set products), and examined to obtain the final product.

### EMBODIMENT 11 (not according to the invention)

### Preparation of Food (Drink)

(1) 600 g *Herba Houttuyniae*, 1000 g *Caulis Lonicerae Japonicae*, 1800 g *Radix Isatidis*, 80 g *Rhizoma Dryopteris Crassirhizomatis*, 404 g *Radix et Rhizoma Sophorae Tonkinensis*, 200 g *Radix Angelicae Dahuricae*, 100 g *Herba Taraxaci*, and 100 g *Herba Andrographiswere* weighed, washed thoroughly, mashed, and then added to alkali solution containing 2%-3% (based on the weight of the raw materials) NaOH and 0.3% sodium hexametaphosphate. The raw materials were heated in the solution at 85 °C for 5 minutes and then fished out, followed by rinsing with running water until the surface turned neutral.
(2) The raw materials were cut into small flakes and then further mashed into a slurry by electric masher. The obtained slurry was transferred to a stainless steel container, adding 2% (based on the weight of the slurry) edible phosphoric acid, and then heated in a water bath holding at 75 °C for 70 minutes while stirring constantly.
(3) The obtained slurry was neutralized with edible sodium bicarbonate, while cooling by water and stirring constantly. The slurry was then filtered and dried by squeezing.
(4) 6% of (of the liquid weight) active carbon powder was added into the filtrate, which was kept in a water bath at 88 °C for 120 minutes and then filtered to obtain slightly yellow or brown clear filtrate. The filtrate was then adjusted and packaged followed by sterilization. The feed liquid was prepared by mixing the obtained drug liquid, phosphoric acid and citric acid in a weight ratio of 100:3:4. Water was added into the obtained liquid to make the weight ratio of the feed liquid to water reach 1:50.

Finally, the obtained mixture was mixed, filled into bottles and sealed, followed by pasteurization.

### EMBODIMENT 12

### Preparation of Veterinary Drug

400 g *Herba Houttuyniae*, 400 g *Caulis Lonicerae Japonicae*, 200 g *Radix Isatidis,* 100 g *Rhizoma Dryopteris Crassirhizomatis*, 1000 g *Radix et Rhizoma Sophorae Tonkinensis*, 1000 g *Radix Angelicae Dahuricae*, 1000 g *Rhizoma Paridis*, 1000 g *Herba Artemisiae Annuae*, and 1000 g *Rhizoma Iridis Tectori* were weighed and smashed, sieved with 80 mesh sieve, mixed thoroughly to obtain powder. After subpackaged, the said veterinary drug was finally obtained.

### EMBODIMENT 13

### Preparation of Vaccine

1500 g *Herba Houttuyniae*, 400 g *Caulis Lonicerae Japonicae*, 584 g *Radix Isatidis,* 800 g *Rhizoma Dryopteris Crassirhizomatis*, 500 g *Radix et Rhizoma Sophorae Tonkinensis*, and 500 g *Radix Angelicae Dahuricae* were decocted in water and then filtered. The filtrate was concentrated under reduced pressure, followed by adding ethanol and then standing. The concentrated filtrate was filtered and concentrated under reduced pressure until 11 g raw medicine per ml was reached. The concentrate was then diluted with water, refrigerated, filtered, and concentrated under reduced pressure. The solution was separated with macroporous resin which was washed with water, NaOH solution and 55 wt. % ethanol successively and then eluted with 65 wt. % ethanol. The eluate was collected, diluted with water and then adjusted to pH 7.0 by adding L-Arginine. Active carbon was added, heated and removed by filtration. The filtrate was diluted with the same volume of physiological saline to obtain the said vaccine.

### EMBODIMENT 14 (not according to the present invention)

### Preparation Technique of Capsules

2000 g *Herba Houttuyniae*, 200 g of *Caulis Lonicerae Japonicae*, 400 g *Radix Isatidis*, 900 g *Radix et Rhizoma Sophorae Tonkinensis*, and 784 g *Flos Lonicerae Japonicae* were weighed and soaked in 3200 ml water for injection for 1 hour. 3200 ml water for injection was added and decocted twice for 2.5 hours per time. After filtering, the pooled filtrate was recovered under reduced pressure until no ethanol was smelt and then concentrated to obtain a thick paste with a relative density of 1.30. The paste was then dried under reduced pressure at 80 °C for 48-96 hours in a vacuum drying oven, smashed and sieved with 60-80 mesh sieve. The obtained powder was then mixed with adjuvant, sieved with 60 mesh sieved and granulated. The granules were dried by heating, sieved and then mixed with magnesium stearate. The mixture was finally filled into capsules.

### EMBODIMENT 15

### Preparation of Injections of Preventive Drug (not according to the present invention)

484 g *Herba Houttuyniae*, 500 g *Caulis Lonicerae Japonicae*, 1800 g *Radix Isatidis*, and 1500 g *Radix et Rhizoma Sophorae Tonkinensis* were decocted in water and then filtered. The filtrate was concentrated under reduced pressure. Ethanol was then added and left for standing. The solution was filtered and the filtrate was concentrated under reduced pressure until 11 g raw medicine per ml was reached. The concentration was diluted with water, refrigerated, filtered, and concentrated under reduced pressure followed by separating with macroporous resin which was washed with water, NaOH solution and 55 wt. % ethanol successively and then eluted with 65 wt. % ethanol. The eluate was collected, diluted with water and then adjusted to pH 7.0 by adding L-Arginine. Active carbon was added, heated, and then removed by filtration. The filtrate was diluted with the same volume of physiological saline to obtain the said injection of preventive drug.

### EMBODIMENT 16 (not according to the present invention)

### Preparation Technique of Capsules

1600 g *Herba Houttuyniae*, 800 g *Caulis Lonicerae Japonicae*, 800 g *Radix Isatidis*, 600 g *Radix et Rhizoma Sophorae Tonkinensis*, and 484 g *Herba Andrographis* were weighed and soaked in 3200 ml water for injection for 1 hour. 3200 ml water for injection was added and decocted twice for 2.5 hours per time. After filtering, the pooled filtrate was recovered under reduced pressure until no ethanol was smelt and then concentrated to obtain a thick extractum with a relative density of 1.30. The extractum was dried under reduced pressure at 80 °C for 48-96 hours in a vacuum drying oven, smashed, and sieved with 60-80 mesh sieve. The obtained powder was then mixed with adjuvant, sieved with a 60 mesh sieve and granulated. The granules were dried by heating, sieved and mixed with magnesium stearate. The mixture was finally filled into capsules.

### EMBODIMENT 17 (not according to the present invention)

### Preparation Technique of Capsules

1700 g *Herba Houttuyniae*, 1000 g *Caulis Lonicerae Japonicae*, 400 g *Radix Isatidis*, 500 g *Radix et Rhizoma Sophorae Tonkinensis*, and 684 g *Fructus Bruceae* were weighed and soaked in 3200 ml water for injection for 1 hour. 3200 ml water for injection was added and decocted twice for 2.5 hours per time. After filtering, the pooled filtrate recovered under reduced pressure until no ethanol was smelt and then concentrated to obtain a thick extractum with a relative density of 1.20. The extractum was dried under reduced pressure at 80 °C for 48-96 hours in a vacuum drying oven, smashed and sieved with 60-80 mesh sieve. The obtained powder was then mixed with adjuvant, sieved with a 60 mesh sieve and granulated. The granules were dried by heating, sieved, and mixed with magnesium stearate. The mixture was finally filled into capsules.

### EMBODIMENT 18

### Preparation Technique of Capsules

1500 g *Herba Houttuyniae*, 1000 g *Caulis Lonicerae Japonicae*, 100 g *Radix Isatidis*, 800 g *Rhizoma Dryopteris Crassirhizomatis*, 500 g *Radix et Rhizoma Sophorae Tonkinensis*, and 384 g *Radix Angelicae Dahuricae* were weighed, soaked in 3200 ml water for injection for 1 hour. 7200 ml water for injection was added and decocted twice for 1 hour per time. After filtration, the pooled filtrate recovered under reduced pressure until no ethanol was smelt and then concentrated to obtain a thick extractum with a relative density of 1.0 (60°C). The extractum was then dried under reduced pressure at 70 °C for 36 hours in a vacuum drying oven, smashed and sieved with 60-80 mesh sieve.

The obtained powder was then mixed with adjuvant, sieved and granualated. The granules were dried by heating, sieved with a 20 mesh sieve, and then mixed with magnesium stearate. The mixture was finally filled into capsules.

### EMBODIMENT 19 (not according to the present invention)

### Preparation Technique of Syrup

200 g o*Herba Houttuyniae*, 200 g *Caulis Lonicerae Japonicae*, 1800 g *Radix Isatidis,* and 1500 g *Radix et Rhizoma Sophorae Tonkinensis* were weighed and extracted by steam distillation to obtain the volatile oil for use. After that, six- to eight-fold amount of water was added and decocted for three times for 1 hour per time. After filtration, the pooled filtrate was concentrated under reduced pressure (40 °C) to obtain suitable amount of the solution Following precipitation at low temperature for 24 hours. The supernatant was collected and mixed with the volatile oil, syrupus simplex, and preservative (sodium benzoate), and distilled water was then added to result in a final volumn of 1000 ml. The solution was mixed thoroughly, subpackaged and sterilized by flowing steam for 30 minutes.

The following Embodiments 20-23 further illustrate that the traditional Chinese medicine composition of the present invention have advantages, comparing the existing anti-swine influenza drugs, by verifying the cytological and zoological experiment results for the traditional Chinese medicine composition of the present invention.

### EMBODIMENT 20

The protective effect of the traditional Chinese medicine composition prepared in Embodiment 5 on the chickens infected with swine influenza virus.

The chickens tested in this experiment were healthy 50-day-old yellow chickens which were purchased from the Experimental Chicken Farm of Sun Yat-Sen University. The drug examined in this embodiment was the traditional Chinese medicine composition of the present invention according to Embodiment 5. One of the control drugs was rimantadine which was purchased from Zhejiang Yukang Pharmaceuticals Co., Ltd. The other control drug was prepared according to China Patent No. 200510011432.3. The virus stain was H1N1 serum subtype of swine influenza virus (SIV) (1.0 x 10⁻⁶).

### Experimental method:

190 healthy yellow chickens were divided into 5 groups randomly: 30 chickens for the normal control group while 40 chickens for each of the other groups. The allantoic fluid of chick embryos containing the swine influenza virus was diluted to a concentration of 0.5 x 10⁻⁵. Each chicken was inoculated by intraperitoneal injection with 0.2 ml of the dilution. On the same day when the chickens were infected with AIV, the drugs were administered to chickens of each group, respectively, while the normal control group and infected control group were fed with physiological saline. The drugs or physiological saline was administered once per day continuously for 11 days. Group A was the normal control group. Group B was treated with the traditional Chinese medicine composition of this embodiment (10 mg/day). Group C was treated with rimantadine (10 mg/day), one of the control drugs. Group D was treated with the drug prepared according to China Patent No. 200510011432.3 (10 mg/day), the other control drug. Group E was the infected control group. The results of the experiment are shown in Table 1.

**Table 1**

| **Group** | **Number of Chickens** | **Treatment** | **Number of Death** | **Death Rate** | **Effective Rate** |
|---|---|---|---|---|---|
| A | 30 | Non-infection | 0 | -- | -- |
| B | 40 | Infection followed by drug administration | 1 | 2.5% | 97.5% |
| C | 40 | Infection followed by drug administration | 25 | 62.5% | 37.5% |
| D | 40 | Infection followed by drug administration | 18 | 45% | 55% |
| E | 40 | Infection but without drug administration | 32 | 80% | -- |

Table 1 shows that the traditional Chinese medicine composition of the present invention has excellent effect on protecting the chickens infected with the swine influenza virus, better than the effect of the drug prepared according to China Patent No. 200510011432.3 and the traditional treating drug rimantadine.

### EMBODIMENT 21

Protective Effect of the Traditional Chinese Medicine Composition Prepared in Embodiment 2 on the Chickens Infected with the Swine Influenza Virus.

The chickens used in this experiment were healthy 50-day-old yellow chickens which were purchased from the Experimental Chicken Farm of Sun Yat-sen University. The drug examined in this embodiment was the traditional Chinese medicine composition of present invention according to Embodiment 2. One of the control drugs was rimantadine which was purchased from Zhejiang Yukang Pharmaceuticals Co., Ltd. The other control drug was prepared according to China Patent No. 200510011432.3. The virus strain was the H1N1 subtype serum swine influenza virus (1.0 x 10⁻⁶).

### Experimental method:

150 healthy yellow chickens were divided into 5 groups randomly: 30 chickens for each group. The allantoic fluid of chick embryos containing the swine influenza virus was diluted to a concentration of 0.5 x 10⁻⁵. Each chicken was inoculated by intraperitoneal injection with 0.2 ml of the dilution. On the same day when the chickens were infected with AIV, the drugs were administered to chickens of each group, respectively, while the normal control group and the infected control group were fed with physiological saline. The drugs or physiological saline was injected once per day for successive 7 days. Group F was the normal control group. Group G was treated with the traditional Chinese medicine composition of this embodiment (10 mg/day). Group H was treated with rimantadine (10 mg/day), one of the control drugs. Group I was treated with the drug prepared according to China Patent No. 200510011432.3 (10 mg/day). Group J was the infected control group. The results are shown in Table 2.

**Table 2**

| **Group** | **Number of Chickens** | **Treatment** | **Number of Death** | **Death Rate** | **Effective Rate** |
|---|---|---|---|---|---|
| F | 30 | Non-infection | 0 | -- | -- |
| G | 30 | Infection followed by drug administration | 2 | 6.7% | 93.3% |
| H | 30 | Infection followed by drug administration | 19 | 63.3% | 36.6% |
| I | 30 | Infection followed by drug administration | 15 | 50% | 50% |
| J | 30 | Infection but without drug administration | 27 | 90% | -- |

Table 2 shows that the traditional Chinese medicine composition of the present invention has excellent effect on protecting the chickens infected with the swine influenza virus, better than the effect of the drug prepared by China Patent No. 200510011432.3 and the traditional treating drug rimantadine.

### EMBODIMENT 22

Assay of Treating Viral Pneumonia Caused by H1N1 Influenza A Virus.

The mice used in the experiment were Kunming mice provided by Laboratory of Experimental Animal in Harbin Medical University. The mice were injected with H₁ virus to create mouse model. Prescription amounts of the injections of traditional Chinese medicine composition were used in this experiment.

### Experimental method:

There were 17 mice in each experimental groups and control groups. Control Group 1 was intraperitoneally injected with physiological saline (15 ml/kg); Control Group 2 was intraperitoneally injected with Zedoray Turmeric oil injection (15 ml/kg) which is often used as anti-virus drug; Control Group 3 was intraperitoneally injected with Shuanghuanglian injection (15 ml/kg) which is often used to treat pneumonia. Experimental Group 1 (not according to the present invention) was intraperitoneally injected with the injection solution (15 ml/kg) which was made from *Herba Houttuyniae*, *Caulis Lonicerae Japonicae*, *Radix Isatidis*, and *Rhizoma Dryopteris Crassirhizomatis*; Experimental Group 2 (not according to the present invention) was intraperitoneally injected with the injection solution (15 ml/kg) which was made from *Herba Houttuyniae*, *Caulis Lonicerae Japonicae*, *Radix Isatidis*, *Rhizoma Dryopteris Crassirhizomatis*, and *Radix Rhizoma Glycyrrhizae*; Experimental Group 3 (according to the present invention) was intraperitoneally injected with the injection solution (15 ml/kg) which was made from *Herba Houttuyniae*, *Caulis Lonicerae Japonicae*, *Radix Isatidis*, *Rhizoma Dryopteris Crassirhizomatis*, *Radix et Rhizoma Sophorae Tonkinensis*, and *Radix Angelicae Dahuricae.* All drugs were administrated continuously for 10 days. Mice in all groups were sacrificed after 11-day of administration and the samples were collected for testing. The results are shown in Table 3.

**Table 3**

| **Group** | **Significantly Effective** | **Effective** | **Not effective** | **Dead** |
|---|---|---|---|---|
| Experimental Group 1 | 10 | 4 | 3 | 1 |
| Experimental Group 2 | 8 | 3 | 6 | 6 |
| Experimental Group 3 | 11 | 5 | 1 | 0 |
| Control Group 1 | 0 | 3 | 14 | 12 |
| Control Group 2 | 3 | 5 | 9 | 8 |
| Control Group 3 | 2 | 4 | 11 | 8 |

| | | | | |
|---|---|---|---|---|
| Note: "Significantly effective" means that the patient condition was improved markedly, body temperature returned to normal and inflammation was eliminated; "Effective" means the patient condition was improved, body temperature returned to normal, inflammation was slightly reduced and mild cough occurred. "Not effective" means the patient condition was not improved or was even aggravated. | | | | |

Table 3 shows that, comparing with Control Groups 1, 2 and 3, the traditional Chinese medicine composition used in Experimental Groups 1, 2, and 3 had a significant therapeutic effect against the H1N1 influenza A virus, including improving pulmonary circulation, clearing platelets aggregation, eliminating pulmonary inflammation, and repairing lung damage. Therein, the medicine composition tested in Experimental Group 3 has better effect than those tested in Experimental Groups 1 and 2.

### EMBODIMENT 23

*In vitro* Cytological Experiment of the Traditional Chinese Medicine Composition Prepared in Embodiment 7 against Swine Influenza Virus

### I. Materials

### 1. Testing drugs

The drug (KBD-SC) tested in this experiment was the traditional Chinese medicine composition of the present invention according to Embodiment 7 (4.7 g raw medicine per ml of extractum). The control drug was the raw material of Tamiflu, that is, Oseltamivir phosphate, purchased from Roche Pharmaceutical Company.

### 2. Cells:

The cells used in this experiment were MDCK cells (Madin-Darby Canine Kidney Cells), provided by Microbiology Department, Medical School, The University of Hong Kong.

### 3. Viruses:

The viruses used in this experiment were A/California/4/2009 and A/Hong Kong/xx/2008, provided by Microbiology Department, Medical School of The University of Hong Kong.

### II. Methods:

1. Cytotoxicity assay: The drug concentration (CC50) at which 50% of cells were subjected to pathological changes was determined according to the standard Mosmann method (1983).
   a. Preparation of the drug medicine sample: the extractum of the traditional Chinese medicine composition was diluted with 1x MEM culture medium to a final concentration of 200 g/ml. After mixing thoroughly, the mixture was centrifuged at 2000 rpm for 5 min, and the supernatant was collected as the testing drug.
   b. Preparation of 24-well plates of MDCK cell culture: MDCK cells of 70-80% cell saturation were incubated in 24-well plates at 37 °C / 5% CO₂.
   c. The testing drug solution of 200 g/ml was diluted to a concentration of 100 mg/ml, 50 mg/ml, 25 mg/ml, 12.5 mg/ml, and 6.25 mg/ml.
   d. The testing drug solution of each concentration was added to the MDCK plates at 500 µl per well, respectively, and then the MDCK plates were incubated in an incubator at 37 °C / 5% CO₂.
   e. The growth status of MDCK cells was observed continually for two days to determine the initial concentration of the testing drug.
   f. Two concentrations of the testing drug, namely 10mg/ml and 5mg/ml were selected in the experiments
2. Antiviral assay:
   a. The virus culture of 100 TCID5 was prepared according to the TCID50 virus titer.
   b. 5 ml of the testing drug solution of the two different concentrations, 10mg/ml and 5mg/ml, was diluted, respectively; while the concentrations of Tamiflu were 100 µmol/ml and 10 µmol/ml.
   c. 1 ml of virus culture was mixed with 1 ml of the testing drug solution and the control drug of different concentrations, respectively, and then incubated at room temperature for 30 minutes.
   d. The mixture of virus and drug was added into the 24-well MDCK cell culture plates at 120 µl/well, respectively, in duplicates and the plates were then incubated in an incubator at 37 °C / 5% CO₂ for 1 hour.
   e. The mixtures of virus and drug were removed and different concentrations of the testing drug were added to the plate, respectively, at 1ml/well in duplicate. The MDCK cell culture plates were incubated in an incubator at 37 °C / 5% CO₂.
   f. The pathological changes of the cells were observed continually for three days. Photographs were taken on Day 2 and Day 3, and in the meantime the supernatants were examined by Real-Time PCR. The results are shown in Table 4.

**Table 4**

| | **Day 2** | | **Day 3** | |
|---|---|---|---|---|
| Concentration of The Testing Drug | 10 mg | 5 mg | 10 mg | 5 mg |
| SC Number of Detectable Viruses / Total Number | 0/2 | 0/2 | 1/2 | 2/2 |
| Concentration of Tamiflu | 100 µmol/ml | 10 µmol/ml | 100 µmol/ml | 10 µmol/ml |
| Number of Detectable viruses / Total Number | 0/2 | 1/2 | 2/2 | 2/2 |

Conclusion: The KBD-SC extractum of the traditional Chinese medicine composition of the present invention prepared according to Embodiment 7 substantially inhibited replication of the virus on Day 2 and was still effective on Day 3.

## Claims

1. Use of a traditional Chinese medicine composition in preparation of drugs and/or health-care products for preventing or treating swine influenza in human or animal, wherein the swine influenza is influenza A caused by H1N1, and wherein said traditional Chinese medicine composition comprises active ingredients made from *Herba Houttuyniae*, *Caulis Lonicerae Japonicae*, *Radix Isatidis*, *Radix et Rhizoma Sophorae Tonkinensis*, *Rhizoma Dryopteris Crassirhizomatis* and *Radix Angelicae Dahuricae*, and wherein the weight percentages of *Herba Houttuyniae*, *Caulis Lonicerae Japonicae*, *Radix Isatidis*, *Radix et Rhizoma Sophorae Tonkinensis*, *Rhizoma Dryopteris Crassirhizomatis* and *Radix Angelicae Dahuricae* are: *Herba Houttuyniae* 4.7 wt. %-58.4 wt. %, *Caulis Lonicerae Japonicae 4.7* wt. %-46.7 wt. %, *Radix Isatidis* 2.3 wt. %-42.0 wt. %, *Radix et Rhizoma Sophorae Tonkinensis* 1.2 wt. %-35.0 wt. %, *Rhizoma Dryopteris Crassirhizomatis* 1.9 wt. %-37.3 wt. %, and *Radix Angelicae Dahuricae* 1.2 wt. %- 35.0 wt. %.

2. Use of a traditional Chinese medicine composition in preparation of drugs and/or health-care products for preventing or treating swine influenza in human or animal, wherein the swine influenza is influenza A caused by H1N1, and wherein said traditional Chinese medicine composition comprises active ingredients and pharmaceutically acceptable carriers, wherein the active ingredients are made from: *Herba Houttuyniae* 9.3 wt. %-46.7 wt. %, *Caulis Lonicerae Japonicae* 9.3 wt. %-37.3 wt. %, *Radix Isatidis* 4.7 wt. %-35.0 wt. %, *Rhizoma Dryopteris Crassirhizomatis* 2.3 wt. %-28.0 wt. %, *Radix et Rhizoma Sophorae Tonkinensis* 2.3 wt. %-23.3 wt. %, *Radix Angelicae Dahuricae* 2.3 wt. %- 23.3 wt. %, *Rhizoma Paridis* 2.3 wt. %-23.3 wt. %, *Herba Artemisiae Annuae* 2.3 wt. %-23.3 wt. %, and *Rhizoma Iridis Tectori* 2.3 wt. %-23.3 wt. %.

3. The use according to Claim 2, wherein the active ingredients are made from: *Herba Houttuyniae* 14.0 wt. %-35.0 wt. %, *Caulis Lonicerae Japonicae* 11.7 wt. %-23.3 wt. %, *Radix Isatidis* 9.3 wt. %-21.0 wt. %, *Rhizoma Dryopteris Crassirhizomatis* 7.0 wt. %-18.7 wt. %, *Radix et Rhizoma Sophorae Tonkinensis* 4.7 wt. %-11.7 wt. %, *Radix Angelicae Dahuricae* 4.7 wt. %-11.7 wt. %, *Rhizoma Paridis* 4.7 wt. %-11.7 wt. %, *Herba Artemisiae Annuae* 4.7 wt. %-11.7 wt. %, and *Rhizoma Iridis Tectori* 4.7 wt. %-11.7 wt. %.

4. The use according to Claim 2 or 3, wherein the active ingredients are made from: *Herba Houttuyniae* 21.4 wt. %, *Caulis Lonicerae Japonicae* 17.9 wt. %, *Radix Isatidis* 14.3 wt. %, *Rhizoma Dryopteris Crassirhizomatis* 10.7 %, *Radix et Rhizoma Sophorae Tonkinensis* 7.1 wt. %, *Radix Angelicae Dahuricae* 7.1 wt. %, *Rhizoma Paridis* 7.1 wt. %, *Herba Artemisiae Annuae* 7.1 wt. %, and *Rhizoma Iridis Tectori* 7.1 wt. %.

5. The use according to any one of Claims 1-4, wherein the traditional Chinese medicine composition is made into a pharmaceutically acceptable dosage form.

6. The use according to any one of Claims 1-5, wherein the traditional Chinese medicine composition is made into granule dosage form.

7. The use according to any one of Claims 1-6, wherein the traditional Chinese medicine composition according to any of Claims 1-6 is prepared by a method comprising the following steps:
(1) weighing the raw materials for use;
(2) decocting Herba Houttuyniae in water for 10-40 minutes and filtering the decoction; concentrating the filtrate to obtain a clear paste with a relative density of 1.05 and cooling down the paste;
(3) adding ethanol to the clear paste obtained in Step (2) until the content of ethanol reaches 50-80%, followed by standing for 24 hours and filtering; recovering the ethanol from the filtrate; concentrating the filtrate to obtain a clear paste with a relative density of 1.25;
(4) decocting other raw materials in water twice, 2 hours each time, and filtering the decoction; concentrating the filtrate until the relative density reaches 1.20; filtering and concentrating the concentrate again to obtain a clear paste with a relative density of 1.10-1.25;
(5) mixing the clear pastes obtained in Step (3) and Step (4) and concentrating continuously to obtain a thick paste with a relative density of or greater than 1.26; adding adjuvant to obtain the traditional Chinese medicine composition.

## Patentansprüche

1. Verwendung einer traditionellen chinesischen Medizinzusammensetzung zur Herstellung von Arzneimitteln und/oder Gesundheitspflegeprodukten zur Vorbeugung oder Behandlung von Schweinegrippe bei Mensch oder Tier, wobei die Schweinegrippe Influenza A ist, die durch H1N1 verursacht wird, und wobei die traditionelle chinesische Medizinzusammensetzung Wirkstoffe umfasst, die aus *Herba Houttuyniae, Caulis Lonicerae Japonicae, Radix Isatidis, Radix et Rhizoma Sophorae Tonkinensis, Rhizoma Dryopteris Crassirhizomatis* und *Radix Angelicae Dahuricae* hergestellt sind, und wobei die Gewichtsprozentsätze von *Herba Houttuyniae, Caulis Lonicerae Japonicae, Radix Isatidis, Radix et Rhizoma Sophorae Tonkinensis, Rhizoma Dryopteris Crassirhizomatis* und *Radix Angelicae Dahuricae* sind: *Herba Houttuyniae* 4,7 Gew.-% bis 58,4 Gew.-%, *Caulis Lonicerae Japonicae* 4,7 Gew.-% bis 46,7 Gew.-%, *Radix Isatidis* 2,3 Gew.-% bis 42,0 Gew.-%, *Radix* et *Rhizoma Sophorae Tonkinensis* 1,2 Gew.-% bis 35,0 Gew.-%, *Rhizoma Dryopteris Crassirhizomatis* 1,9 Gew.-% bis 37,3 Gew.-% und *Radix Angelicae Dahuricae* 1,2 Gew.-% bis 35,0 Gew.-%.

2. Verwendung einer traditionellen chinesischen Medizinzusammensetzung zur Herstellung von Arzneimitteln und/oder Gesundheitspflegeprodukten zur Vorbeugung oder Behandlung von Schweinegrippe bei Mensch oder Tier, wobei die Schweinegrippe Influenza A ist, die durch H1N1 verursacht wird, und wobei die traditionelle chinesische Medizinzusammensetzung Wirkstoffe und pharmazeutisch annehmbare Träger umfasst, wobei die Wirkstoffe hergestellt sind aus: *Herba Houttuyniae* 9,3 Gew.-% bis 46,7 Gew.-%, *Caulis Lonicerae Japonicae* 9,3 Gew.-% bis 37,3 Gew.-%, *Radix Isatidis* 4,7 Gew.-% bis 35,0 Gew.-%, *Rhizoma Dryopteris Crassirhizomatis* 2,3 Gew.-% bis 28,0 Gew.-%, *Radix et Rhizoma Sophorae Tonkinensis* 2,3 Gew.-% is 23,3 Gew.-%, *Radix Angelicae Dahuricae* 2,3 Gew.-% bis 23,3 Gew.-%, *Rhizoma Paridis* 2,3 Gew.-% bis 23,3 Gew.-%, *Herba Artemisiae Annuae* 2,3 Gew.-% bis 23,3 Gew.-% und *Rhizoma Iridis Tectori* 2,3 Gew.-% bis 23,3 Gew.-%.

3. Verwendung nach Anspruch 2, wobei die Wirkstoffe hergestellt sind aus: *Herba Houttuyniae* 14,0 Gew.-% bis 35,0 Gew.-%, *Caulis Lonicerae Japonicae* 11,7 Gew.-% bis 23,3 Gew.-%, *Radix Isatidis* 9,3 Gew.-% bis 21,0 Gew.-%, *Rhizoma Dryopteris Crassirhizomatis* 7,0 Gew.-% bis 18,7 Gew.-%, *Radix et Rhizoma Sophorae Tonkinensis* 4,7 Gew.-% bis 11,7 Gew.-%, *Radix Angelicae Dahuricae* 4,7 Gew.-% bis 11,7 Gew.-%, *Rhizoma Paridis* 4,7 Gew.-% bis 11,7 Gew.-%, *Herba Artemisiae Annuae* 4,7 Gew.-% bis 11,7 Gew.-% und *Rhizoma Iridis Tectori* 4,7 Gew.-% bis 11,7 Gew.-%.

4. Verwendung nach Anspruch 2 oder 3, wobei die Wirkstoffe hergestellt sind aus: *Herba Houttuyniae* 21,4 Gew.-%, *Caulis Lonicerae Japonicae* 17,9 Gew.-%, *Radix Isatidis* 14,3 Gew.-%, *Rhizoma Dryopteris Crassirhizomatis* 10,7 Gew.-%, *Radix et Rhizoma Sophorae Tonkinensis* 7,1 Gew.-%, *Radix Angelicae Dahuricae* 7,1 Gew.-%, *Rhizoma Paridis* 7,1 Gew.-%, *Herba Artemisiae Annuae* 7,1 Gew.-% und *Rhizoma Iridis Tectori* 7,1 Gew.-%.

5. Verwendung nach einem der Ansprüche 1 bis 4, wobei die traditionelle chinesische Medizinzusammensetzung in einer pharmazeutisch annehmbaren Dosierungsform hergestellt wird.

6. Verwendung nach einem der Ansprüche 1 bis 5, wobei die traditionelle chinesische Medizinzusammensetzung in einer Granulatdosierungsform hergestellt wird.

7. Verwendung nach einem der Ansprüche 1 bis 6, wobei die traditionelle chinesische Medizinzusammensetzung gemäß einem der Ansprüche 1 bis 6 durch ein Verfahren hergestellt wird, welches die folgenden Schritte umfasst:
(1) Wiegen der zu verwendenden Rohstoffe;
(2) Auskochen von Herba Houttuyniae in Wasser für 10 bis 40 Minuten und Filtrieren des Dekokts; Konzentrieren des Filtrats, um eine klare Paste mit einer relativen Dichte von 1,05 zu erhalten, und Abkühlen der Paste;
(3) Zugabe von Ethanol zu der in Schritt (2) erhaltenen klaren Paste bis der Ethanolgehalt 50 bis 80% erreicht, gefolgt von 24 Stunden Stehenlassen und Filtrieren; Rückgewinnen des Ethanols aus dem Filtrat; Konzentrieren des Filtrats, um eine klare Paste mit einer relativen Dichte von 1,25 zu erhalten;
(4) zweimaliges Auskochen der anderen Rohstoffe in Wasser von jeweils 2 Stunden und Filtrieren des Dekokts; Konzentrieren des Filtrats bis die relative Dichte 1,20 erreicht; Filtrieren und erneutes Konzentrieren des Konzentrats, um eine klare Paste mit einer relativen Dichte von 1,10 bis 1,25 zu erhalten;
(5) Mischen der in Schritt (3) und Schritt (4) erhaltenen klaren Pasten und kontinuierliches Konzentrieren, um eine dicke Paste mit einer relativen Dichte von oder mehr als 1,26 zu erhalten; Zugabe eines Hilfsmittels, um die traditionelle chinesische Medizinzusammensetzung zu erhalten.

## Revendications

1. Utilisation d'une composition de médecine traditionnelle chinoise pour la préparation de médicaments et/ou de produits de santé pour la prévention ou le traitement de la grippe porcine chez l'homme ou l'animal, la grippe porcine étant la grippe A causée par le H1N1, et où ladite composition médicinale traditionnelle chinoise comprend des ingrédients actifs à base des éléments suivants: *Herba Houttuyniae*, *Caulis Lonicerae Japonicae*, *Radix Isatidis*, *Radix et Rhizoma Sophorae Tonkinensis*, *Rhizoma Dryopteris Crassirhizomatis* et *Radix Angelicae Dahuricae*, et où les pourcentages en poids de *Herba Houttuyniae, Caulis Lonicerae Japonicae, Radix Isatidis, Radix et Rhizoma Sophorae Tonkinensis, Rhizoma Dryopteris Crassirhizomatis* et *Radix Angeli cae Dahuricae* sont: *Herba Houttuyniae* 4,7 % en poids - 58,4 % en poids, *Caulis Lonicerae Japonicae* 4,7 % en poids - 46,7 % en poids, *Radix Isatidis* 2,3 % en poids - 42,0 % en poids, *Radix et Rhizoma Sophorae Tonkinensis* 1,2 % en poids - 35,0 % en poids, *Rhizoma Dryopteris Crassirhizomatis* 1,9 % en poids - 37,3 % en poids *et Radix Angelicae Dahuricae* 1,2 % en poids - 35,0 % en poids.

2. Utilisation d'une composition de médecine traditionnelle chinoise pour la préparation de médicaments et/ou de produits de santé pour la prévention ou le traitement de la grippe porcine chez l'homme ou l'animal, la grippe porcine étant la grippe A causée par le H1N1, et où ladite composition médicinale traditionnelle chinoise comprend des ingrédients actifs et des véhicules acceptables pharmaceutiquement, où les ingrédients actifs sont choisis parmi les éléments suivants: *Herba Houttuyniae* 9,3 % en poids - 46,7 % en poids, *Caulis Lonicerae Japonicae* 9,3 % en poids - 37,3 % en poids, *Radix Isatidis* 4,7 % en poids - 35,0 % en poids, *Rhizoma Dryopteris Crassirhizomatis* 2,3 % en poids - 28,0 % en poids, *Radix et Rhizoma Sophorae Tonkinensis* 2,3 % en poids - 23,3 % en poids, *Radix Angelicae Dahuricae* 2,3 % en poids - 23,3 % en poids, *Rhizoma Paridis* 2,3 % en poids - 23,3 % en poids, *Herba Artemisiae Annuae* 2,3 % en poids - 23,3 % en poids et *Rhizoma Iridis Tectori* 2,3 % en poids - 23,3 % en poids.

3. Utilisation selon la revendication 2, où les ingrédients actifs sont choisis parmi les éléments suivants: *Herba Houttuyniae* 14,0 % en poids - 35,0 % en poids *Caulis Lonicerae Japonicae* 11,7 % en poids - 23,3 % en poids, *Radix Isatidis* 9,3 % en poids - 21,0 % en poids, *Rhizoma Dryopteris Crassirhizomatis* 7,0 % en poids - 18,7 % en poids, *Radix et Rhizoma Sophorae Tonkinensis* 4,7 % en poids - 11,7 % en poids, *Radix Angelicae Dahuricae* 4,7 % en poids - 11,7 % en poids, *Rhizoma Paridis* 4,7 % en poids - 11,7 % en poids, *Herba Artemisiae Annuae* 4,7 % en poids - 11,7 % en poids et *Rhizoma Iridis Tectori* 4,7 % en poids - 11,7 % en poids.

4. Utilisation selon les revendications 2 ou 3, où les ingrédients actifs sont choisis parmi les éléments suivants: *Herba Houttuyniae* 21,4 % en poids, *Caulis Lonicerae Japonicae* 17,9 % en poids, *Radix Isatidis* 14,3 % en poids, *Rhizoma Dryopteris Crassirhizomatis* 10,7 % en poids, *Radix et Rhizoma Sophorae Tonkinensis* 7,1 % en poids, *Radix Angelicae Dahuricae* 7,1 % en poids, *Rhizoma Paridis* 7,1 % en poids, *Herba Artemisiae Annuae* 7,1 % en poids et *Rhizoma Iridis Tectori* 7,1 % en poids.

5. Utilisation selon l'une quelconque des revendications 1 à 4, dans laquelle la composition médicinale traditionnelle chinoise est transformée en une forme posologique pharmaceutiquement acceptable.

6. Utilisation selon l'une quelconque des revendications 1 à 5, dans laquelle la composition médicinale traditionnelle chinoise est transformée en une forme posologique galénique.

7. Utilisation selon l'une quelconque des revendications 1 à 6, dans laquelle la composition médicinale traditionnelle chinoise selon l'une quelconque des revendications 1 à 6 est préparée par un procédé comprenant les étapes suivantes:
(1) peser les matières premières à utiliser;
(2) décocter Herba Houttuyniae dans l'eau pendant 10 à 40 minutes et filtrer la décoction;
concentrer le filtrat pour obtenir une pâte transparente d'une densité relative de 1,05 et refroidir la pâte;
(3) ajouter de l'éthanol à la pâte transparente obtenue à l'étape (2) jusqu'à ce que la teneur en éthanol atteigne 50 à 80%, étape suivie d'une période de repos de 24 heures et d'une filtration; récupérer l'éthanol du filtrer; concentrer le filtrat pour obtenir une pâte transparente de densité relative de 1,25;
(4) décocter d'autres matières premières dans de l'eau deux fois, 2 heures à chaque fois, et filtrer la décoction; concentrer le filtrat jusqu'à ce que la densité relative atteigne 1,20; filtrer et concentrer le concentrat à nouveau pour obtenir une pâte transparente d'une densité relative de 1,10-1,25;
(5) mélanger les pâtes transparentes obtenues aux étapes (3) et (4) et concentrer en continu pour obtenir une pâte épaisse de densité relative supérieure ou égale à 1,26; ajouter un adjuvant pour obtenir la composition médicinale traditionnelle chinoise.
